# EUROPEAN PATENT SPECIFICATION

(11) **EP 2 335 687 B1**
(45) Date of publication and mention of the grant of the patent: **24.05.2017**
(21) Application number: 09804144.5
(22) Date of filing: 17.08.2009
(51) Int. Cl.: A61K 9/127, C12N 15/88

(54) **USE OF MONOOLEIN AS A NEW AUXILIARY LIPID IN LIPOFECTION**
VERWENDUNG VON MONOOLEIN ALS NEUES HILFSLIPID IN DER LIPOFEKTION
UTILISATION DE MONOOLÉINE COMME NOUVEAU LIPIDE ADJUVANT DANS LA LIPOFECTION

(30) Priority: 18.08.2008 PT 10415808
(43) Date of publication of application: 22.06.2011
(73) Proprietor: Universidade do Minho, 4704-320 Braga (PT)
(72) Inventor: CUNHA DIAS REAL OLIVEIRA, Maria Elisabete, P-4710-057 Braga (PT); GOMES COUTINHO, Paulo José, P-4710-057 Braga (PT); FERNANDES PEREIRA COUTINHO, Olga Maria, P-4710-057 Braga (PT); FERREIRA CASTRO GOMES, Andreia, P-4710-057 Braga (PT); PEDRA AMORIM CASAL, Margarida Paula, P-4710-057 Braga (PT); P. N. SILVA, João, P-4710-057 Braga (PT)
(74) Representative: Ferreira, Maria Silvina
(86) International application number: PCT/IB2009/053619
(87) International publication number: WO 2010/020935

(56) References cited:
- EP-A2- 1 713 446
- NEVES SILVA J P ET AL: "Characterization of Monoolein-Based Lipoplexes Using Fluorescence Spectroscopy" JOURNAL OF FLUORESCENCE, KLUWER ACADEMIC PUBLISHERS-PLENUM PUBLISHERS, NE, vol. 18, no. 2, 23 December 2007 (2007-12-23), pages 555-562, XP019576252 ISSN: 1573-4994
- ZUHORN INGE S ET AL: "Phase behavior of cationic amphiphiles and their mixtures with helper lipid influences lipoplex shape, DNA translocation, and transfection efficiency" BIOPHYSICAL JOURNAL, vol. 83, no. 4, October 2002 (2002-10), pages 2096-2108, XP002577962 ISSN: 0006-3495

## Description

### Field of Invention

The genotypic transformation of a cell implies a fenotypic transformation leading to a permanent hereditary change of the DNA and its expression profile. This phenomenon can be artificially obtained through the insertion of exogenous genetic material in the cell (transfection). The present disclosure relates to a new cationic lipid formulation (lipofection agent), through the conjugation of the neutral lipid monooleoyl-rac-glycerol (Monoolein) with the cationic tensioactives derived from Dioctadecyldimethylammonium (DODAX) family (Dioctadecyldimethylammonium Bromide (DODAB), Dioctadecyldimethylammonium Chloride (DODAC) and Dioctadecyldimethylammonium Phosphate (DODAP)), with application both in molecular biology (*in vitro* application) and in the health area in gene therapy (*in vivo* application).

### Background of the Invention

To the phenotypic modification of a cell, induced by a permanent hereditary change of the DNA and its expression profile, it is given the name of transformation. This phenomenon can be artificially obtained through the insertion of exogenous genetic material in the cell (transfection) and reveals itself of extreme importance both in the investigation field in Molecular Biology (in vitro application) and in Gene Therapy health area (in vivo application).

The several transfection methods currently available have been frequently grouped in two main groups: viral methods (recombinant viruses) and non-viral methods (complexation of DNA through lipids or polymers, coprecipitation of DNA with calcium phosphate, electroporation, gene gun, or DNA microinjection) (Maslov et al., 2002).

Table I summarizes the main advantages and disadvantages associated with these two types of transfection systems.

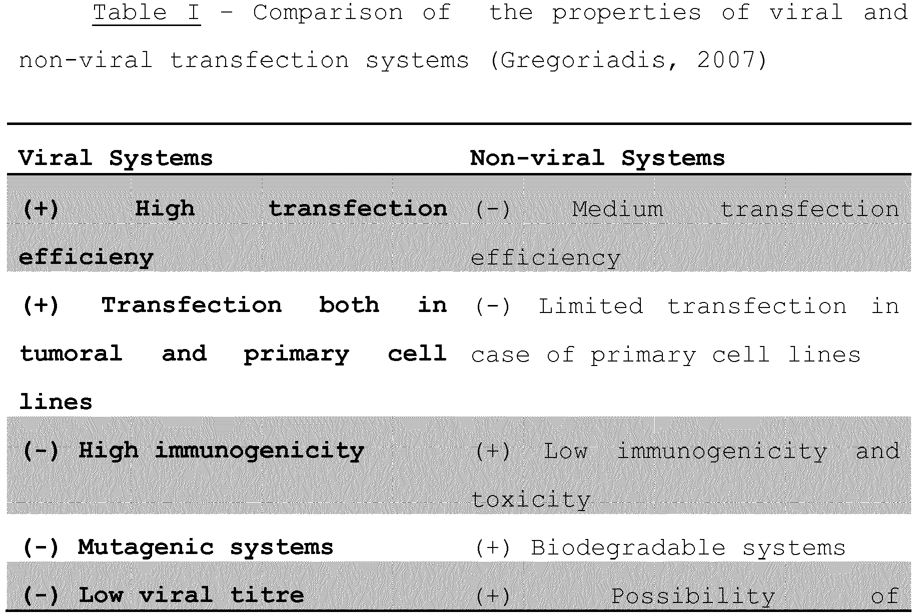

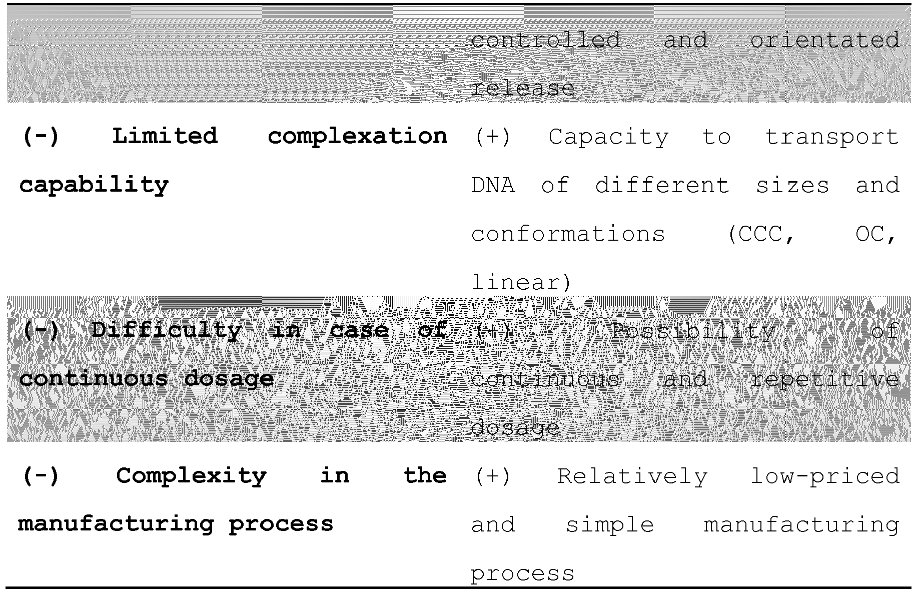

Among the large group of non-viral methods, special relevance has been given in the last decades to the lipofection method, which involves the use of cationic liposomes for the complexation and release of exogenous DNA inside the cell. Felgner and his colleagues described for the first time in 1987 the use of lipidic molecules with positively charged chemical groups for gene transfection in culture cell lines (Felgner et al.,1987). Unlike pharmacological compounds that are transported within lipidic systems, plasmid DNA is not encapsulated in the liposomes interior, but remains tightly condensed due to the activity of small cationic vesicles that cover total-or partially the plasmid, originating specialized structures that became later known as lipoplexes, which formation is highly dependent on the electrostatic attraction between the negative DNA phosphate backbone and the cationic lipid positive head-groups (Labat-Moleur et al., 1996; Meager, 1999).

The development of new lipids for application in gene therapy occurs essentially through the empirical testing of new compounds that have never been used before for that purpose, each one of them being analysed for a specific application. After the initial breakthrough given by Felgner and his collaborators, a first investigation phase resulted in the discovery of numerous molecules, as can be observed in several published documents: US5279833 (Rose, 1994),US6936469 (G. deJong, S.L. Vanderbyl, 1998).New developments continue to be made in the synthesis of new cationic lipids for transfection, as referred in the documents US5651981 (Asley et al, 1997), WO2005033289 (R. MacDonald, L. Wang, 2005), where it is described the application of mixtures of the synthetic cationic lipids [1,2-dioleoyl-sn-glycero-3-ethylphosphocholine (EDOPC), 1,2-dilauroyl-sn-glycero-3-ethylphosphocholine (EDLPC), and 1,2-dimyristol-sn-glycero-3-ethylphosphocholine (EDMPC)] in transfection systems with no indication of presence of helper lipids. Another document US7067697 (Xiang Gao, 2006), presents mixtures of several cationic lipids combined with functional bioactive agents for pharmaceutical applications, where 1,2-dioleoyl-sn-glycero-3-phosphotidylethanolamine (DOPE) and cholesterol are identified as helper lipids.

It has been stated that the inclusion of neutral helper lipids in the lipoplex formulation promotes an increase in DNA release rate and in transfection efficiency, either by promoting the fusion process of larger lipoplexes with the cell membrane or by enhancing the endocytosis process of smaller dimensions lipoplexes (M.R. Almofti et al., 2003).

However, depending on the origin and properties of the cell type used, the same lipofection system may present very different transfection efficiencies, which has justified the permanent need to search new formulations adapted to each cell type.

Of the several documents published until now, the most frequently cited helper lipids are DOPE, cholesterol and its derivatives [US5888821 (R. Reszka, 1999) and US7001614 (N. Smyth-Templeton e G.N. Pavlakis, 2006)].
Therefore, even if the helper lipids promote, in general, the transfection process, they are one of the several factors that affect transfection efficiency, since the cell type itself to transfect is decisive for the process efficiency. In addition, other parameters such as the lipoplex size and the complexes structure type of the may be also important for the development of a successful transfection system, because they limit the biofunctionality, biocompatibility and the toxicity of said systems. All these factors help to explain the reason why a high DNA complexation efficiency is not necessarily associated to a high transfection rate.

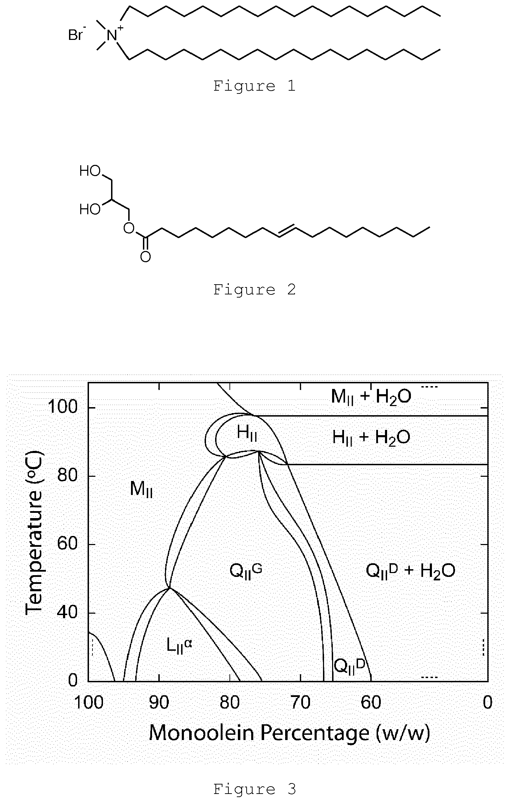

Recently, the synthetic tensioactive Dioctadecyldimethylammonium Bromide (DODAB) (Fig. 1) was used by the authors of the present invention in conjugation with a low-cost natural lipid (Monoolein) to develop vesicular systems which have demonstrated to efficiently complex commercial DNA (Silva et al., 2008). Nevertheless, similarly to what was already verified in many other vesicular systems (C. Esposito et al., 2006) and of the previously mentioned, high DNA complexation efficiency is not necessarily associated to a high transfection rate.

Monoolein, 1-monooleoyl-rac-glycerol (MO) (Fig. 2), is a neutral amphiphilic lipid of natural origin that owns the distinctiveness of possessing two inverted bicontinuous cubic phases, even in excess of H₂O (< 90% w/w), as can be observed by its phase diagram, represented in Fig. 3 (Lipowsky et al, 1995). The existence of several non-lamellar phases, together with its low-cost commercial value, indicates that Monoolein could be a potential attractive helper lipid.

The structure of the lipid/DNA complexes strongly depends on the proportion between cationic lipid:DNA, as well as on the proportion between cationic lipid: neutral lipid (May et al., 2004). In addition, the lipoplex formation itself depends on the liposomes preparation method and on the procedure that is used to complex the DNA by the cationic vesicles (Simões et al., 2005). It is also recognized that the order of addition of lipids and DNA has a critical effect on the physical properties and biological activity of the resulting lipoplexes (Tranchant et al., 2004). As follows, the effectiveness of monoolein in DNA complexation (Silva et al., 2008) does not make obvious its application in lipofection.

Until now, the use of monoolein as helper lipid in lipofection systems hasn't been described, despite its low-cost commercial value and its reduced citotoxicity, as is now proposed in the present invention. Monoolein has only been referred as being part of formulations which use encapsulate DNA in liposomes, and not by lipofection through direct DNA complexation [WO2003/057190 (2003)]. The application of monoolein as a helper also brings the additional advantage that it can be applied in different cell types.

The document US6074667 (Paavo Kinnunem et al; 1998) mentions transfection methods using as non-viral vectors cationic lipids derived from sphingosines, using DOPE as first helper lipid and diacylglycerol derivatives as secondary helper lipid in KK-1 cell line. Unlike monoolein which belongs to the monoacylglycerol group, the diacylglycerols have two hydrophobic tails, being therefore similar to the common lipid helpers as DOPE.

### Brief Description of the Figures

**Fig.1** - Chemical representation of Dioctadecyldimethylammonium Bromide (DODAB) molecule.
**Fig.2** **-** Chemical representation of 1-monooleoyl-rac-glycerol (Monoolein) molecule.
**Fig.3** **-** Phase Diagram of 1-monooleoyl-rac-glycerol (Monoolein) containing different lyotropic phases: lamellar structure (L_{α}; L_{β}); inverted hexagonal non-lamellar structure (H_{II}) and inverted cubic non-lamellar structure (Q_{II}).
**Fig.4** **-** Graphical representation of transfection efficiency comparison of different lipid/DNA formulations in 293T cell line.
**Fig. 5** **-** Citoxicity comparison of different lipid/DNA formulations in 293T cell line.

### Brief Description of the Invention

The present disclosure describes the application of cationic lipidic systems composed of cationic surfactants derived from Dioctadecyldimethylammonium (DODAX) and the neutral tensioactive 1-monooleoyl-rac-glycerol (Monoolein, MO), for the complexation and transport of DNA, as well as a means of genetic transformation for cells. Monoolein, 1-monooleoyl-rac-glycerol (MO), is a neutral amphiphilic lipid of natural origin that owns the distinctiveness of possessing two inverted bicontinuous cubic phases, even in excess of water (< 90% w/w), as can be observed by its phase diagram, represented in Fig. 3 (Lipowsky et al, 1995). The existence of several non-lamellar phases, together with its low-cost commercial value, indicate that monoolein could be a potential attractive helper lipid in lipofection systems.

The DNA complexation, determined by the electrostatic attraction between the positively charged ammonium groups of the synthetic surfactant Dioctadecyldimethylammonium (DODAX) and the negatively charged phosphate backbone of the plasmid DNA, causes the condensation of the structure, thus originating lipid/DNA complexes also known as lipoplexes, that in this case also contain neutral lipid Monoolein.

The inclusion of Monoolein in the lipoplex formulation grants it completely distinctive features, due to the fact that monoacylglycerols present a distinct curvature pattern from the cationic surfactant used.
The decrease in structural rigidity of the Dioctadecyldimethylammonium DODAX vesicles, caused by the inclusion of Monoolein, increases the lateral mobility of the lipidic chain, which in the end promotes the cell membrane/lipoplex interaction, thus favouring the transfection process.

### Detailed Description of the Invention

Being applied monoolein:cationic lipid molar ratios between 0.1 and 0.9, an application example of the invention consists in the use of a formulation with the cationic lipid Dioctadecyldimethylammonium Bromide DODAB:lipid helper (Monoolein) molar ratio 2:1 and distinct charge ratios (+/-) (2:1 and 4:1) of the possibilities (of 1:1 to 4:1), corresponding to different concentrations of the positive ammonium groups of Dioctadecyldimethylammonium Bromide (DODAB) for the same concentration of negative phosphate groups (DNA).

The relative efficiency of the systems (in terms of their transfection effectiveness and citotoxicity level) was determined by direct comparison with the commercial lipofection system Lipofectamin®, in the conditions proposed by the manufacturer (Invitrogen).

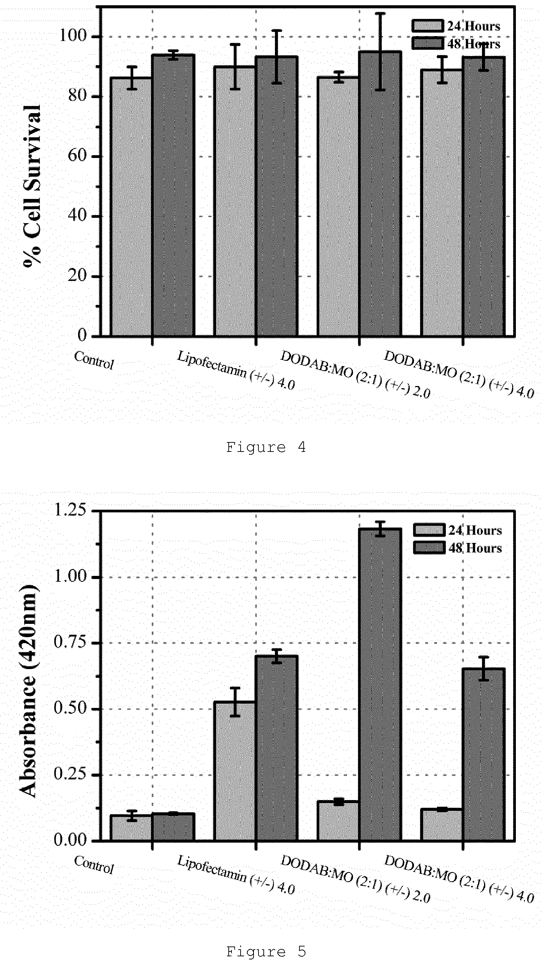

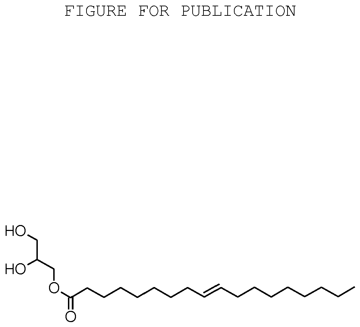

The transfection efficiency was determined through the measuring of the β-galactosidase reporter gene activity (Absorbance at 420nm) (Fig. 4). The citotoxicity level was determined through the measuring of the Lactate Dehydrogenase activity (Absorbance at 340nm) (Fig. 5).

In the presented example, the use of two different charge ratios (+/-) (2:1 and 4:1) for the DODAB:MO (2:1) system was due to the need of determining the minimum lipid concentration that would allow to obtain the maximum transfection rate without representing an increase of toxicity to the cells themselves.

For the tested lipidic formulation, although it was expected that charge ratio (+/-) 4:1 presented higher transfection efficiency than charge ratio (+/-) 2:1 (because of the higher concentration of the lipofection agent), that was not experimentally observed (Fig. 4), probably due to the citotoxicity increase observed at 48h (5% to 7% respectively at charge ratios (+/-) 2:1 and 4:1) (Fig. 5). It must also be referred that the commercial system (Lipofectamin) presents a higher toxicity level (7%) than the DODAB:MO (2:1) system, at charge ratio (+/-) 2:1.

Nevertheless, the demonstration that the DODAB:Monoolein (2:1) formulation at distinct charge ratios (+/-) present comparable transfection efficiencies (C.R. (+/-) 2:1) or even superior (C.R. (+/-) 4:1) to the commercial system, indicate that this model in the invention is effective at variable concentrations of lipid and DNA.

This versatility can reveal itself useful in the application of this lipofection system in other cell lines where the charge ratios (+/-) 2:1 are less efficient than others and where other transfection conditions are required to increase the process efficiency.

### Preparation of mixed cationic vesicles

For preparing the mixed liposome solutions, defined volumes from the stock solutions of Dioctadecyldimethylammonium Bromide DODAB and Monoolein in ethanol (20 mM) were injected, under vigorous vortexing, to an aqueous buffer solution at 70 °C (30 mM Tris-HCl). A final lipid concentration (DODAB + Monoolein) of 1 mM was obtained for the cationic lipid (DODAB):helper lipid (Monoolein) molar ratio of 2:1.

### Preparation of cationic lipoplexes

The cationic lipoplexes were prepared through the addition of defined volumes of the mixed liposome solutions (variable concentrations of positive ammonium groups) to a constant volume of DNA solution (fixed concentration of negative phosphate units = number of wells × 1µg DNA/well). Different volumes of Opti-MEM I were used to complete a total constant volume of solution in the several formulations and charge ratios (+/-) tested (100µL/well). The resulting lipoplexes were incubated at room temperature during 30min without agitation.

The genetic material introduced in the cell was was the pSV-β-gal (Invitrogen) plasmid, which was amplified by *Escherichia coli DHB4* competent cell line. The extraction and purification of the DNA was made using the "Wizard® Plus Midipreps DNA Purification System" extraction kit, commercialized by Promega. The final DNA concentration (1.75µg/µL) was measured through the absorbance at 260nm in a Shimadzu UV-3101-PC spectrophotometer.

### Cell Culture

For transfection and citotoxicity determination assays, the 293T cell line cells were cultivated in T75cm³ flasks with DMEM medium supplemented with L-glutamin (4mM), sodium bicarbonate (1.5 g/L), glucose (4.5 g/L), fetal bovine serum (10% v/v) and antibiotics (1 uni/ml). The cells were regularly subcultivated between passage numbers 18 and 24, being always kept at confluences lower than 90% (37°C and 5% CO₂).

### Lipofection

Twelve hours prior to transfection, each T75cm³ flask was washed once with PBS (1X) solution and the cells were released through the addition of trypsin solution (0.05%) (3 minutes incubation at 37°C and 5% CO₂). The number of viable cells was quantified in a hemocytometer through the trypan blue exclusion method. Cells were ressuspended in an adequate DMEM medium to obtain a density of 2×10⁵ cells/well, and were transferred to 24-Multiwell plates at a final volume of 500 µl/well (approximately 50-80% confluence on the day of the transfection).

In the day of transfection, the culture medium was replaced by fresh DMEM medium, and the recently prepared lipoplexes (see section "Preparation of cationic lipoplexes") were homogeneously added to each well, in a quantity that did not exceed 20% of the final volume (1 µg DNA/well). The cells were then once more incubated at 37°C and 5% CO₂, for a 24h-48h period. The transfection efficiency was determined through the determination of β-galactosidase reporter gene activity level. The citotoxicity level was determined by measuring the intra and extracelular Lactate Dehydrogenase (LDH) enzyme in the analysed samples.

### Determination of transfection efficiency (β-galactosidase activity assay)

In order to determine the transfection efficiency, recently incubated cells (24h-48h) were washed twice with PBS (1X) solution and disrupted with lysis buffer solution (RLB 1X) for 15 minutes. The cells were then recollected and centrifuged for 2 min at 14000rpm to remove cellular debris. β-galactosidase enzyme activity was determined in the several samples (in arbitrary units of absorbance, a.u.) with the "β-galactosidase Enzyme Assay System with Reporter Lysis Buffer" expression kit, accordingly with the manufacturer instructions (1 µl of cellular extract + 49 µl of RLB (1X) solution + 50 µl of Assay (2X) buffer solution + 150 µl of sodium carbonate solution, to stop the colorimetric reaction), with absorbance reading at 420nm in a 96 Multi-well plate with a SpectraMax 340PC microplate reader.

### Determination of citotoxicity level (Lactate Dehydrogenase activity assay)

For determination of the citotoxicity level in the analyzed systems, the intra and extracellular amounts of the Lactate Dehydrogenase enzyme were quantified. The collection of the culture medium in each well allowed the measurement of the extracellular LDH. To obtain the value of intracellular LDH, the wells were washed with 500 µl of Tris-HCl buffer solution (15mM), followed by mechanic scraping and ultrasound lysis of the cell samples for 3 periods of 30seg sonication. Both samples (intra- and extracellular) obtained were centrifuged for 1 minute at 13000rpm to remove cellular debris. The intracellular activity of LDH enzyme was determined [10 µl of intracellular sample + 250 µl of NADH (0.31 mM) solution + 10 µl of pyruvate (8.96 mM) solution to stop the colorimetric reaction] in a 96 Multi-well plate with a SpectraMax 340PC microplate reader (absorbance at 340nm).

The extracellular activity of the LDH enzyme was also determined [40 µl of extracellular sample + 250 µl of NADH (0.31 mM) solution + 10 µl of pyruvate (8.96 mM) solution to stop the colorimetric reaction] in a 96 Multi-well plate with a SpectraMax 340PC microplate reader (absorbance at 340nm).

The balance between the intra and extracellular activities of the LDH enzyme, allows the determination of the cell survival rate.

### REFERENCES

### Articles

M.A. Maslov, E.I. Syicheva, N.G. Morozova, G.A. Serebrennikova, (2002), "Cationic Amphiphiles of Both Lipid and Non-lipid Nature in Gene Therapy", (2000), Russian Chemical Bulletin, v. 49, n. 3, pp. 385-401.
G. Gregoriadis, (2007), "Liposome Technology - II - Entrapment of Drugs and Other Materials Into Liposomes", 3rd Edition, Editions I. Healthcare, New York (USA), pp. 424.
P.L. Felgner, T.R. Gadek, M. Holm, R. Roman, H.W. Chan, M. Wenz, J.P. Northrop, R.M. Ringold, M. Danielson, (1987), "Lipofection: a highly efficient, lipid-mediated DNA-transfection procedure, Proceedings of the National Academy of Sciences USA, n. 84, pp. 7413-7417.
F. Labat-Moleur, A.M. Steffan, C. Brisson, H. Perron, O. Feugeas, P. Furstenberger, F. Oberling, E. Brambilla, J.P. Behr, (1996), "An electron microscopy study into the mechanism of gene transfer with lipopolyamines", Gene Therapy, n. 11, pp. 1010-1017.
A. Meager, (1999), "Gene Therapy Technologies, Applications and Regulations", 1st Edition, Editions J.W.S. Limited, New York (USA), pp. 438.
S.W. Hui, et al., (1996), "The Role Of Helper Lipids In Cationic Liposome-Mediated Gene Transfer", Biophysical Journal, n. 71, pp. 590-599.
P.L. Felgner, Y.I. Tsai, L. Sukhu, C.J. Wheeler, M. Manthorpe, J. Marshall, S.H. Cheng, (1995), "Improved cationic lipid formulations for in vivo gene therapy", Ann.N. Y. Acad. Sci., n. 772, pp. 126-139.
H. Farhood, R. Bottega, R.M. Epand, L. Huang, (1992), "Effect of cationic cholesterol derivatives on gene transfer and protein kinase C activity", Biochim. Biophys. Acta, n. 2, pp. 239-246.
G.J. Nabel, D. Gordon, D.K. Bishop, B.J. Nickoloff, Z.Y. Yang, A. Arnga, M.J. Cameron, E.G. Nabel, A.F. Chang, (1996), "Immune response in human melanoma after transfer of an allogeneic class I major histocompatibility complex gene with DNA-liposome complexes", Proc. Natl. Acad. Sci. USA, n. 26, pp. 15388-15393.
Y. Liu, D. Liggitt, W. Zhong, C. Ti, K. Gaensler, R. Debs, (1995), "Cationic liposome mediatedintravenous gene delivery", J. Biol. Chem, n. 42, pp. 24864-24870.
R.I. Mahato, K. Kawabata, Y. Takakura, M. Hashida, (1995), "In vivo disposition characteristics of plasmid DNA complexed with cationic liposomes", J. Drug Targeting, n. 2, pp. 149-157.
N. Oudrhiri, J.P. Vigneron, M. Peuchmaitr, T. Leclerc, J.M. Lehn, P. Lehn, (1997), "Gene transfer by guanidinium-cholesterol cationic lipids into airway epithelial cells in vitro and in vivo", Proc. Natl. Acad. Sci. USA, n. 5, pp. 1651-1656.
P.C.A. Barreleiro, (2001), "Equilibrium and Kinetic Studies of the Binding of DNA to Cationic Liposomes", Ph.D. thesis, Physical Chemistry Department, Center for Chemistry and Chemical Engineering, Lund University, Sweden.
J.P. Neves Silva, P.J.G. Coutinho, M.E.C.D. Real Oliveira, (2008), "Characterization of Monoolein-Based Lipoplexes Using Fluorescence Spectroscopy", Journal of Fluorescence, pp. 1-8.
R. Lipowsky, E. Sackmann, (1995), "Structure and Dynamics of Membranes", 2nd Edition, Elsevier Editions, New York, USA, pp. 957.
S. May, A. Ben-Shaul, (2004), "Modeling of cationic lipid-DNA complexes", Curr. Med. Chem., n. 11, pp. 151-167.
S. Simões, A. Filipe, H. Faneca, M. Mano, N. Penacho, N. Düzgünes, M.P. Lima, (2005), "Cationic liposomes for gene delivery", Expert Opin. Drug Deliv, n. 2, pp. 1-19.
I. Tranchant, et al., (2004), "Physicochemical Optimisation of Plasmid Delivery by Cationic Lipids", The Journal of Gene Medicine, n. 6, pp. S24-S35.
M.R. Almofti et al., (2003), "Cationic Liposome Mediated Gene Delivery: Biophysical study and Mechanism of Internalization", Archives of Biochemistry and Biophysics, n. 410, pp. 246-253.
C. Esposito et al., (2006), "The Analysis of serum on structure, size and toxicity of DDAB-DOPE and DC-Chol-DOPE lipoplexes Contributes to Explain Their Differebt Transfection Efficiency", Colloids and Surfaces B: Biointerfaces, n. 53, pp. 187-192.

### Patents

[A] J.K. Rose, (1994), "Liposomal Transfection of Nucleic Acids into Animal Cells", US5279833, p.1-13.
[B] G. deJong, S.L. Vanderbyl, (2005), "Methods for Delivering Nucleic Acid Molecules Into Cells and Assessment Thereof", US6936469, p 1-39.
[C] R. MacDonald, L. Wang, (2005), "Transfection Reagents", WO2005033289, W.I.P. Organization, p. 1-42.
[D] Xiang Gao, (2006) "Cationic Liposomes for Gene Transfer" US7067697, p.1-44.
[E] Asley et al (1997) "Cationic Phospholipids for transfection" US5651981, p.1-13.
[E] N. Smyth-Templeton, G.N. Pavlakis, (2006), "Liposome Complexes for Increased Systemic Delivery", US7001614, p. 1-23.
[F] R. Reszka, (1999), "Cholesterol Derivative for Liposomal Gene Transfer", US5888821, p. 1-4.
[H] Kinnunem et al (1998), "Liposomal Transfection Method" US6074667, p.1-10.

## Claims

1. Method of production of lipoplexes for transport of nucleic acids in cell transfection, **characterized by** comprising the following steps:
- Preparation of a cationic lipid system containing a monoolein helper lipid and cationic lipid derived from Dioctadecyldimethylammonium (DODAX) wherein the a molar ratio of monoolein:cationic lipid is between 0.1 and 0.9;
- Complexation of the nucleic acid through the addition of a defined volume of the previous cationic lipid system to a constant volume of a nucleic acid solution, at room temperature, for a period equal or superior to 30 minutes, in a culture medium with low bovine serum percentage, forming lipoplexes, wherein the cationic lipid system/nucleic acid charge ratio is between 1:1 and 4:1, preferably 2:1.

2. The method according to the previous claim, **characterized by** the cationic lipid derived from Dioctadecyldimethylammonium (DODAX) being selected from a group comprising Dioctadecyldimethylammonium Bromide (DODAB), Dioctadecyldimethylammonium Chloride (DODAC) and Dioctadecyldimethylammonium Phosphate (DODAP).

3. A method described in claims 1-2, wherein the suitable for use lipoplexes are suitable for use in molecular biology and gene therapy areas, or other healthcare and clinical investigation areas.

## Patentansprüche

1. Methode zur Produktion von Lipoplexe, um Nukleinsäure in Zellentransfektion zu transportieren, **dadurch gekennzeichnet dass** folgende Schritte umfasst:
- Vorbereitung eines kationischen Lipidsystems beinhaltend einen Monoolein-Helferlipid und kationischen Lipid abgeleitet vom Dioctadecyldimethylammonium (DODAX), wobei das Molverhältnis Monoolein:kationischen Lipids zwischen 0.1 und 0.9 liegt;
- Komplex des Nukleinsäure durch Zusatz eines definierten Volumens des vorigen kationischen Lipidsystems gegenüber einem konstanten Volumen einer Nukleinsäurelösung, bei Zimmertemperatur, für einen Zeitraum gleich oder höher als 30 Minuten, in einem Nährboden mit niedrigem Rinderserumprozentsatz, Lipoplexen formend, wobei das Ladunsverhältnis kationisches Lipidsystem/Nukleinsäure zwischen 1:1 und 4:1, vorzugsweise 2:1, liegt.

2. Die Methode gemäß vorigem Anspruch **dadurch gekennzeichnet dass** den kationischen Lipid abgeleitet vom Dioctadecyldimethylammonium (DODAX), ausgewählt von einer Gruppe umfassend Dioctadecyldimethylammoniumbromid (DODAB), Dioctadecyldimethylammoniumchlorid (DODAC) und Dioctadecyldimethylammoniumphosphat (DODAP) ist.

3. Eine Methode gemäß Ansprüche 1-2, wobei die Lipoplexe zur Anwendung in den Bereichen der Molekularbiologie und der Gentherapie oder anderen gesundheitlichen und klinischen Untersuchungsbereichen geeignet sind.

## Revendications

1. Méthode de production de lipoplexes pour le transport d'acides nucléiques dans la transfection de cellule, **caractérisée par** le fait de comprendre les étapes suivantes:
- Préparation d'un système lipide cationique contenant un lipide accessoire de monooléine et un lipide cationique dérivé du Dioctadecyldimethylammonium (DODAX), où le rapport molaire du lipide cationique et de monooléine est compris entre 0.1 et 0.9 ;
- Complexation de l'acide nucléique à travers l'ajout d'un volume défini du précédent système de lipide cationique vers un volume constant de solution d'acide nucléique à température ambiante, pour une période égale ou supérieure à 30 minutes, au sein d'un milieu de culture ayant un faible pourcentage de sérum bovin, formant les lipoplexes, où le système lipide cationique/rapport de charge de l'acide nucléique est compris entre 1:1 et 4:1, et de préférence à 2:1.

2. La méthode conformément à la revendication précédente, **caractérisée par** le lipide cationique dérivé du Dioctadecyldimethylammonium (DODAX), étant sélectionné d'un groupe comprenant du Bromure de Dioctadecyldimethylammonium (DODAB), du Chlorure de Dioctadecyldimethylammonium(DODAC) et du phosphate de Dioctadecyldimethylammonium (DODAP).

3. Méthode décrite aux revendications 1-2, où les lipoplexes sont appropriées à l'usage dans les domaines thérapeutiques de la biologie moléculaire et de la génétique, ou d'autres domaines d'investigation clinique et de la santé.
